# EUROPÄISCHE PATENTSCHRIFT

(11) **EP 2 143 401 B1**
(45) Veröffentlichungstag und Bekanntmachung des Hinweises auf die Patenterteilung: **21.11.2018**
(21) Anmeldenummer: 09162534.3
(22) Anmeldetag: 12.06.2009
(51) Int. Cl.: A61B 5/00, A61B 17/72, A61B 17/80, A61F 2/82

(54) **Implantatsystem mit einem Funktionsimplantat aus abbaubarem Metallmaterial**
Implant system having a functional implant of degradable metal material
Système d'implant avec un implant fonctionnel en matériau métallique dégradable

(30) Priorität: 08.07.2008 DE 102008040253
(43) Veröffentlichungstag der Anmeldung: 13.01.2010
(73) Patentinhaber: BIOTRONIK AG, 8180 Bülach (CH)
(72) Erfinder: Klocke, Björn, 8006 Zürich (CH); Gerold, Bodo, 91225 Zellingen (DE); Fringes, Matthias, 91522 Ansbach (DE)
(74) Vertreter: Keck, Hans-Georg

(56) Entgegenhaltungen:
- EP-A- 0 966 979
- WO-A-01/54617
- WO-A-2008/013879
- US-A1- 2006 271 168

## Beschreibung

Die Erfindung betrifft ein Implantatsystem umfassend ein eine medizinische Funktion erfüllendes Funktionsimplantat, das zumindest teilweise aus einem vom Körper des Implantatträgers abbaubaren Metallmaterial gebildet ist.

Zum Hintergrund der Erfindung ist festzuhalten, dass derartige Implantate beispielsweise in Form von Nägeln, Schrauben oder Platten zur Therapie bei Knochenbrüchen eingesetzt werden. Grundsätzlich soll der im Körper ablaufende Vorgang beim Zusammenwachsen von Bruchenden von Knochen möglichst ungestört und anatomisch regelgerecht von statten gehen. Die üblichste Methode dabei ist die Verwendung eines Ruhigstellungsverbandes in Form einer Gips- oder Kunststoffmanschette. Dieser wird meist bei unkomplizierten Brüchen verwendet. Der Nachteil dieser Methode ist der lange Funktionsverlust der geschädigten Extremität, was zu Muskel- und Knochenrückbildung (Atrophie) bei langer Schonzeit führt.

Um dies zu verhindern und möglichst frühzeitig wieder mit der Rehabilitation beginnen zu können, wird bei Knochenbrüchen zunehmend eine Operation durchgeführt, bei der mithilfe von Nägeln, Schrauben oder Platten eine exaktere Wiederherstellung der Anatomie und eine bessere Fixierung der Fragmente möglich sind. Für ältere Patienten ist dies meist die einzige Möglichkeit, ihre vorherige Selbständigkeit nicht auf lange Zeit oder permanent zu verlieren.

Üblicherweise werden solche Funktionsimplantate zur Stützung, Fixierung und Halterung aus nicht-abbaubaren Metallen, wie medizinischem Stahl oder Titan(-Legierung) hergestellt. Nach dem Zusammenwachsen der Knochenfragmente verbleiben diese Implantate im Körper oder werden im Zuge einer weiteren Operation explantiert.

Im Körper verbleibende Funktionsimplantate können auf Dauer zu Irritationen, wie Entzündungen, führen. Die Explantierung des Implantats führt zu einem zweiten, vermeidbaren Eingriff. WO 01/54617 A1 beschreibt diesbezüglich einen Thrombusfilter.

Um hier Abhilfe zu schaffen, sind beispielsweise aus der US 2008/0015578 A1 orthopädische Implantate bekannt, die zumindest teilweise aus vom Körper des Implantatträgers abbaubaren Metallmaterialien hergestellt sind. Es werden Metalle wie Eisen, Magnesium, Zink, Wolfram sowie Mischungen oder Legierungen daraus eingesetzt.

Aus der US 2006/0052782 A1 ist ein aktives orthopädisches Implantat etwa in Form einer Knochenplatte bekannt, das zumindest einen Mikrochip und einen damit verbundenen Sensor aufweist. Der Sensor spricht auf physikalische Signale vom Implantat oder dem Patientengewebe, wie beispielsweise Temperatur, Druck oder Zugbeanspruchung an. Die vom Sensor erfasste Information wird vom Mikrochip verarbeitet und auf einen außerhalb des Patienten angeordneten Empfänger, wie beispielsweise einen Personalcomputer, übertragen. Damit kann vom medizinischen Betreuungspersonal eine Diagnose beispielsweise der wirksamen Funktionsdauer des Implantats, der Belastung auf die Knochenplatte und möglicher Komplikationen vorgenommen werden, wie sie bei orthopädischen Implantaten in Form von Infektionen, Nicht-Heilung der Bruchstelle und Ermüdung auftreten können. Das Implantat kann darüber hinaus Elektroden auf seiner Oberfläche aufweisen, um den Heilungsprozess der Knochenfraktur zu unterstützen.

Nachteilig bei den Funktionsimplantaten aus einem abbaubaren Metallmaterial ist die Tatsache, dass der Abbauprozess von den jeweiligen Gegebenheiten im Körper des Implantatträgers abhängig ist. Insbesondere kann der Abbauprozess nicht aktiv beeinflusst werden.

Davon ausgehend liegt der Erfindung die Aufgabe zugrunde, ein Implantatsystem der gattungsgemäßen Art anzugeben, bei dem der Abbauprozess, insbesondere der Zeitpunkt des Beginns und die Dauer des Abbauprozesses aktiv beeinflussbar sind.

Diese Aufgabe wird durch ein Implantatsystem mit den Merkmalen des Anspruch 1 gelöst. Demnach ist vorgesehen eine implantierbare Steuereinrichtung zur Steuerung des Abbauverhaltens des Funktionsimplantats mit einer Gegenelektrode zum Funktionsimplantat, die aus einem gegenüber dem Metallmaterial des Funktionsimplantates elektrochemisch edleren Metallmaterial besteht, und mit einer Spannungsquelle zur Bereitstellung einer Polarisationsspannung zwischen Funktionsimplantat und Gegenelektrode zur Steuerung des Degradationsverhaltens des Funktionsimplantats.

Mithilfe dieser Steuereinrichtung ist es möglich, das Abbauverhalten des Funktionsimplantates zielgerichtet zu steuern, indem durch die zwischen Funktionsplantat und Gegenelektrode angelegte Polarisationsspannung das Funktionsimplantat kathodisch geschützt wird und somit nicht oder zumindest langsamer degradiert. Somit kann die Auflösung des Implantates nach dessen Einsetzen zuerst verzögert oder vollständig unterbunden werden, sobald allerdings seine Stütz-, Fixier- oder Haltefunktion erfüllt ist, kann durch Anlegen der Polarisationsspannung mithilfe der Steuereinrichtung die Degradation des Implantates beschleunigt werden.

Der Begriff Funktionsimplantate umfasst im Sinne der vorliegenden Erfindung auch Gefäßstützen, Stents oder Depotimplantate. Anwendungsfelder für die Funktionsimplantate sind auch urologische, intrakranielle, vaskuläre und orthopädische Anwendungen, localdrug-delivery-Anwendungen sowie Anwendungen in der Krebstherapie.

Vorzugsweise ist mindestens eine Referenzelektrode vorgesehen zur Ermittlung der am Funktionsimplantat (5, 6, 15) tatsächlich anlegenden Spannung.

Gemäß einer bevorzugten Ausführungsform weist die Steuereinrichtung eine implantierbare Batterie als Spannungsquelle auf. Eine solche Energiequelle ist zuverlässig und äußerst kostengünstig.

Weitere Varianten des Implantatsystems beziehen sich auf die Auslegung der Gegenelektrode, die separat implantierbar oder durch das Gehäuse der implantierbaren Steuereinrichtung selbst gebildet werden kann. Bei einer separaten Ausbildung der Gegenelektrode kann diese selbst aus einem degradierbaren Metallmaterial bestehen, das allerdings elektrochemisch edler als das Metallmaterial des Funktionsimplantates sein muss. Dann muss die Gegenelektrode nicht mehr explantiert werden, sie degradiert mit der Zeit selbst im Körper des Implantatträgers.

Sofern die Gegenelektrode durch das Gehäuse der Steuereinrichtung bzw. dessen Batterie gebildet wird, ist eine nicht-abbaubare Materialauswahl zu treffen, wie beispielsweise ein Titan-Gehäuse. Wahlweise kann auch eine vollständige oder partielle Beschichtung der Gehäuseoberfläche mit einem nicht-abbaubaren Metall, z.B. Platin, oder einer nicht-abbaubaren Legierung, z.B. Platin-Iridium, erfolgen.

Eine Schaltbarkeit der Spannungsquelle kann gemäß einer bevorzugten Ausführungsform dadurch realisiert werden, dass ein Arzt durch einen ambulanten Eingriff die Leitungsverbindung zwischen der Batterie und der Gegenelektrode trennt oder die Batterie selbst explantiert. Eine komfortablere Betätigung der Steuereinrichtung ist allerdings möglich, wenn diese eine telemetrische Datenübertragungseinheit aufweist, wie diese beispielsweise auf dem Gebiet der Herzschrittmacher eine bereits übliche Technik ist. Über die telemetrische Datenübertragungseinheit lassen sich Steuerdaten zur Steuerung der von der Spannungsquelle erzeugten Polarisationsspannung genau so übertragen, wie Messdaten über den Zustand des Funktionsimplantates, wie beispielsweise dessen Degradationsgrad. Damit ist auch im Zusammenhang mit den erfindungsgemäßen Implantatsystemen ein echtes Home Monitoring realisierbar. Es ist für orthopädische Implantate bisher nicht bekannt, dass deren Zustand und der Zustand der behandelten Stelle auch ohne Besuch beim Arzt verfolgbar und beeinflussbar sind.

Eine ebenfalls unter den allgemeinen Kern der Erfindung fallende Umsetzung ist die Ausführung des Funktionsimplantates als Depotimplantat, das mindestens eine mit einem Therapeutikum gefüllte Depotkammer aufweist. Mithilfe der erfindungsgemäßen Steuereinrichtung kann dann die zumindest teilweise aus einem degradierbaren Metallmaterial bestehende Depotkammer zeitlich gesteuert abgebaut und somit das darin befindliche Therapeutikum zeitlich definiert freisetzen. Durch Verwendung mehrerer Depotkammern und unterschiedlicher Materialien oder Dimensionen für deren Abdeckung kann im Zusammenhang mit einer bestimmten, von der Steuereinheit aufgebrachten Polarisationsspannung ein zeitlich versetztes Freisetzen verschiedener Therapeutika gesteuert werden.

Antiproliferative, antiinflammatorische und/oder antimykotische Wirkstoffe können beispielsweise die aus der folgenden Liste gewählt werden:
Abciximab, Acemetacin, Acetylvismion B, Aclarubicin, Ademetionin, Adriamycin, Aescin, Afromoson, Akagerin, Aldesleukin, Amidoron, Aminoglutethemid, Amsacrin, Anakinra, Anastrozol, Anemonin, Anopterin, Antimykotika, Antithrombotika, Apocymarin, Argatroban, Aristolactam-All, Aristolochsäure, Ascomycin, Asparaginase, Aspirin, Atorvastatin, Auranofin, Azathioprin, Azithromycin, Baccatin, Bafilomycin, Basiliximab, Bendamustin, Benzocain, Berberin, Betulin, Betulinsäure, Bilobol, Bisparthenolidin, Bleomycin, Bombrestatin, Boswellinsäuren und ihre Derivate, Bruceanole A,B und C, Bryophyllin A, Busulfan, Antithrombin, Bivalirudin, Cadherine, Camptothecin, Capecitabin, o-Carbamoylphenoxyessigsäure, Carboplatin, Carmustin, Celecoxib, Cepharantin, Cerivastatin, CETP-Inhibitoren, Chlorambucil, Chloroquinphosphat, Cictoxin, Ciprofloxacin, Cisplatin, Cladribin, Clarithromycin, Colchicin, Concanamycin, Coumadin, C-Type Natriuretic Peptide (CNP), Cudraisoflavon A, Curcumin, Cyclophosphamid, Cyclosporin A, Cytarabin, Dacarbazin, Daclizumab, Dactinomycin, Dapson, Daunorubicin, Diclofenac, 1,11-Dimethoxycanthin-6-on, Docetaxel, Doxorubicin, Dunaimycin, Epirubicin, Epothilone A und B, Erythromycin, Estramustin, Etobosid, Everolimus, Filgrastim, Fluroblastin, Fluvastatin, Fludarabin, Fludarabin-5'-dihydrogenphosphat, Fluorouracil, Folimycin, Fosfestrol, Gemcitabin, Ghalakinosid, Ginkgol, Ginkgolsäure, Glykosid 1a, 4-Hydroxyoxycyclophosphamid, Idarubicin, Ifosfamid, Josamycin, Lapachol, Lomustin, Lovastatin, Melphalan, Midecamycin, Mitoxantron, Nimustin, Pitavastatin, Pravastatin, Procarbazin, Mitomycin, Methotrexat, Mercaptopurin, Thioguanin, Oxaliplatin, Irinotecan, Topotecan, Hydroxycarbamid, Miltefosin, Pentostatin, Pegasparase, Exemestan, Letrozol, Formestan, SMC-Proliferation-Inhibitor-2w, Mitoxanthrone, Mycophenolatmofetil, c-myc-Antisense, β-myc-Antisense, β-Lapachon, Podophyllotoxin, Podophyllsäure-2-ethylhydrazid, Molgramostim (rhuGM-CSF), Peginterferon α-2b, Lanograstim (r-HuG-CSF), Macrogol, Selectin (Cytokinantagonist), Cytokininhibitoren, COX-2-Inhibitor, NFkB, Angiopeptin, monoklonale Antikörper, die die Muskelzellproliferation hemmen, bFGF-Antagonisten, Probucol, Prostaglandine, 1-Hydroxy-11-Methoxycanthin-6-on, Scopolectin, NO-Donoren wie Pentaerythrityltetranitrat und Syndnoeimine, S-Nitrosoderivate, Tamoxifen, Staurosporin, β-Estradiol, α-Estradiol, Estriol, Estron, Ethinylestradiol, Medroxyprogesteron, Estradiolcypionate, Estradiolbenzoate, Tranilast, Kamebakaurin und andere Terpenoide, die in der Krebstherapie eingesetzt werden, Verapamil, Tyrosin-Kinase-Inhibitoren (Tyrphostine), Paclitaxel und dessen Derivate wie 6-α-Hydroxy-Paclitaxel, Taxotere, Kohlensuboxids (MCS) und dessen macrocyclische Oligomere, Mofebutazon, Lonazolac, Lidocain, Ketoprofen, Mefenaminsäure, Piroxicam, Meloxicam, Penicillamin, Hydroxychloroquin, Natriumaurothiomalat, Oxaceprol, β-Sitosterin, Myrtecain, Polidocanol, Nonivamid, Levomenthol, Ellipticin, D-24851 (Calbiochem), Colcemid, Cytochalasin A-E, Indanocine, Nocadazole, S 100 Protein, Bacitracin, Vitronectin-Rezeptor Antagonisten, Azelastin, Guanidylcyclase-Stimulator Gewebsinhibitor der Metallproteinase-1 und 2, freie Nukleinsäuren, Nukleinsäuren in Virenüberträger inkorporiert, DNA- und RNA-Fragmente, Plaminogen-Aktivator Inhibitor-1, Plasminogen-Aktivator Inhibitor-2, Antisense Oligonucleotide, VEGF-Inhibitoren, IGF-1, Wirkstoffe aus der Gruppe der Antibiotika wie Cefadroxil, Cefazolin, Cefaclor, Cefotixin Tobramycin, Gentamycin, Penicilline wie Dicloxacillin, Oxacillin, Sulfonamide, Metronidazol, Enoxoparin, desulfatiertes und N-reacetyliertes Heparin (Hemoparin®), Gewebe-Plasminogen-Aktivator, GpIIb/IIIa-Plättchenmembranrezeptor, Faktor Xₐ-Inhibitor Antikörper, Heparin, Hirudin, r-Hirudin, PPACK, Protamin, Prourokinase, Streptokinase, Warfarin, Urokinase, Vasodilatoren wie Dipyramidol, Trapidil, Nitroprusside, PDGF-Antagonisten wie Triazolopyrimidin und Seramin, ACE-Inhibitoren wie Captopril, Cilazapril, Lisinopril, Enalapril, Losartan, Thioproteaseinhibitoren, Prostacyclin, Vapiprost, Interferon α, β und γ, Histaminantagonisten, Serotoninblocker, Apoptoseinhibitoren, Apoptoseregulatoren wie p65, NF-kB oder Bcl-xL-Antisense-Oligonukleotiden, Halofuginon, Nifedipin, Tocopherol Tranilast, Molsidomin, Teepolyphenole, Epicatechingallat, Epigallocatechingallat, Leflunomid, Etanercept, Sulfasalazin, Etoposid, Dicloxacyllin, Tetracyclin, Triamcinolon, Mutamycin, Procainimid, Retinolsäure, Quinidin, Disopyrimid, Flecainid, Propafenon, Sotolol, natürliche und synthetisch hergestellte Steroide wie Inotodiol, Maquirosid A, Ghalakinosid, Mansonin, Streblosid, Hydrocortison, Betamethason, Dexamethason, nichtsteroidale Substanzen (NSAIDS) wie Fenoporfen, Ibuprofen, Indomethacin, Naproxen, Phenylbutazon und andere antivirale Agentien wie Acyclovir, Ganciclovir und Zidovudin, Clotrimazol, Flucytosin, Griseofulvin, Ketoconazol, Miconazol, Nystatin, Terbinafin, antiprozoale Agentien wie Chloroquin, Mefloquin, Quinin, des weiteren natürliche Terpenoide wie Hippocaesculin, Barringtogenol-C21-angelat, 14- Dehydroagrostistachin, Agroskerin, Agrostistachin, 17-Hydroxyagrostistachin, Ovatodiolide, 4,7-Oxycycloanisomelsäure, Baccharinoide B1, B2, B3 und B7, Tubeimosid, Bruceantinoside C, Yadanzioside N, und P, Isodeoxyelephantopin, Tomenphantopin A und B, Coronarin A,B,C und D, Ursolsäure, Hyptatsäure A, Iso-Iridogermanal. Maytenfoliol, Effusantin A, Excisanin A und B, Longikaurin B, Sculponeatin C, Kamebaunin, Leukamenin A und B, 13,18-Dehydro-6-alpha-Senecioyloxychaparrin, Taxamairin A und B, Regenilol, Triptolid, des weiteren Cymarin, Hydroxyanopterin, Protoanemonin, Cheliburinchlorid, Sinococulin A und B, Dihydronitidin, Nitidinchlorid, 12-beta-Hydroxypregnadien 3,20-dion, Helenalin, Indicin, Indicin-N-oxid, Lasiocarpin, Inotodiol" Podophyllotoxin, Justicidin A und B, Larreatin, Malloterin, Mallotochromanol, Isobutyrylmallotochromanol, Maquirosid A, Marchantin A, Maytansin, Lycoridicin, Margetin, Pancratistatin, Liriodenin, Bispsrthenolidin, Oxoushinsunin, Periplocosid A, Ursolsäure, Deoxypsorospermin, Psycorubin, Ricin A, Sanguinarin, Manwuweizsäure, Methylsorbifolin, Sphatheliachromen, Stizophyllin, Mansonin, Streblosid, Dihydrousambaraensin, Hydroxyusambarin, Strychnopentamin, Strychnophyllin, Usambarin, Usambarensin, Liriodenin, Oxoushinsunin, Daphnoretin, Lariciresinol, Methoxylariciresinol, Syringaresinol, Sirolimus (Rapamycin), Somatostatin, Tacrolimus, Roxithromycin, Troleandomycin, Simvastatin, Rosuvastatin, Vinblastin, Vincristin, Vindesin, Teniposid, Vinorelbin, Tropfosfamid, Treosulfan, Tremozolomid, Thiotepa, Tretinoin, Spiramycin, Umbelliferon, Desacetylvismion A, Vismion A und B, Zeorin.

Als weiteres Therapeutikum wäre hier die Gruppe der Therapeutika gegen Krebs zu nennen.

Der Wirkstoff kann in reiner Form oder zusammen mit einem Polymer aufgebracht werden. Für die Aufbringung werden bevorzugt Tauch- oder Sprühverfahren eingesetzt.

Ferner kann der antiproliferative, antiinflammatorische und/oder antimykotische Wirkstoff in einer Polymermatrix eingebettet werden oder sich unter oder auch auf einer Polymermatrix befinden.

Zudem kann sich noch eine weitere polymere biostabile oder bioabbaubare Schicht zusätzlich zu der Polymermatrix auf dem Implantat befinden. Auch diese Schicht kann einen antiproliferativen, antiinflammatorischen und/oder antimykotischen Wirkstoff enthalten, der gleich oder verschieden von dem Wirkstoff in der Polymermatrix sein kann.

Bevorzugte Wirkstoffe, welche sich auf der Oberfläche des Implantats befinden können, werden ausgewählt aus der Gruppe der antiinflammatorischen Wirkstoffe und insbesondere der Cortikosteroide. Als biostabile oder bioabbaubare Polymere und/oder Polymere für die Polymermatrix können folgende Substanzen eingesetzt werden:
Polyvalerolactone, Poly-ε-Decalactone, Polylactonsäure, Polyglycolsäure Polylactide, Polyglycolide, Copolymere der Polylactide und Polyglycolide, Poly-ε-caprolacton, Polyhydroxybuttersäure, Polyhydroxybutyrate, Polyhydroxyvalerate, Polyhydroxybutyrate-covalerate, Poly(1,4-dioxan-2,3-dione), Poly(1,3-dioxan-2-one), Poly-para-dioxanone, Polyanhydride, Polymaleinsäureanhydride, Polyhydroxymethacrylate, Fibrin, Polycyanoacrylate, Polycaprolactondimethylacrylate, Poly-β-Maleinsäure Polycaprolactonbutylacrylate, Multiblockpolymere aus Oligocaprolactondiole und Oligodioxanondiole, Polyetherestermultiblockpolymere aus PEG und Polybutylenterephtalat, Polypivotolactone, Polyglycolsäuretrimethylcarbonate Polycaprolactonglycolide, Poly(γ-ethylglutamat), Poly(DTH-Iminocarbonat), Poly(DTE-co-DT-carbonat), Poly(Bisphenol A-iminocarbonat), Polyorthoester, Polyglycolsäuretrimethylcarbonate, Polytrimethylcarbonate Polyiminocarbonate, Poly(N-vinyl)-Pyrrolidon, Polyvinylalkohole, Polyesteramide, glycolierte Polyester, Polyphosphoester, Polyphosphazene, Poly[p-carboxyphenoxy)propan], Polyhydroxypentansäure, Polyanhydride, Polyethylenoxidpropylenoxid, weiche Polyurethane, Polyurethane mit Aminosäurereste im Backbone, Polyetherester wie das Polyethylenoxid, Polyalkenoxalate, Polyorthoester sowie deren Copolymere, Lipide, Carrageenane, Fibrinogen, Stärke, Kollagen, protein-basierende Polymere, Polyaminosäuren, synthetische Polyaminosäuren, Zein, Polyhydroxyalkanoate, Pectinsäure, Actinsäure, Carboxymethylsulfat, Albumin, Hyaluronsäure, Chitosan und seine Derivate, Heparansulfate und seine Derivate, Heparine, Chondroitinsulfat, Dextran, β-Cyclodextrine, Copolymere mit PEG und Polypropylenglycol, Gummi arabicum, Guar, Gelatine, Collagen Collagen-N-Hydroxysuccinimid, Lipide, Phospholipide, Polyacrylsäure, Polyacrylate, Polymethylmethacrylat, Polybutylmethacrylat, Polyacrylamid, Polyacrylonitrile, Polyamide, Polyetheramide, Polyethylenamin, Polyimide, Polycarbonate, Polycarbourethane, Polyvinylketone, Polyvinylhalogenide, Polyvinylidenhalogenide, Polyvinylether, Polyisobutylene, Polyvinylaromaten, Polyvinylester, Polyvinylpyrollidone, Polyoxymethylene, Polytetramethylenoxid, Polyethylen, Polypropylen, Polytetrafluorethylen, Polyurethane, Polyetherurethane, Silicon-Polyetherurethane, Silicon-Polyurethane, Silicon-Polycarbonat-Urethane, Polyolefin-Elastomere, Polyisobutylene, EPDM-Gummis, Fluorosilicone, Carboxymethylchitosane, Polyaryletheretherketone, Polyetheretherketone, Polyethylenterephtalat, Polyvalerate, Carboxymethylcellulose, Cellulose, Rayon, Rayontriacetate, Cellulosenitrate, Celluloseacetate, Hydroxyethylcellulose, Cellulosebutyrate, Celluloseacetatbutyrate, Ethylvinylacetat-copolymere, Polysulfone, Epoxyharze, ABS-Harze, EPDM-Gummis, Silicone wie Polysiloxane, Polydimethylsiloxane, Polyvinylhalogene und Copolymere, Celluloseether, Cellulosetriacetate, Chitosane und Copolymere und/oder Mischungen der vorgenannten Polymere.

Die Vorteile der Erfindung lassen sich wie folgt zusammenfassen:
- Es sind keine zusätzlichen chirurgischen Eingriffe zur Explantation des Funktionsimplantates notwendig.
- Es verbleiben keine Rückstände im Körper.
- Die Degradation des Funktionsimplantates wird definiert gesteuert. Insbesondere kann sie so verlangsamt oder erst dann gestartet werden, wenn sie wirklich physiologisch sinnvoll oder notwendig ist.
- Mithilfe der Erfindung ist auch eine zeitlich kontrollierte, lokale Ausbringung von Wirkstoffen im Körper realisierbar.

Weitere Merkmale, Einzelheiten und Vorteile der Erfindung ergeben sich aus der nachfolgenden Beschreibung eines Ausführungsbeispiels anhand der beigefügten Zeichnungen. Es zeigen:
- Fig. 1 bis 3: schematische Darstellungen einer Körperpartie mit Knochenfraktur und Implantatsystem in unterschiedlichen Ausführungsformen,
- Fig. 4: ein Ersatzschaltbild für das Implantatsystem,
- Fig. 5: eine schematische Darstellung eines Depotimplantates,
- Fig. 6: Schaubild eines kathodischen Schutzsystems, sowie
- Fig. 7: eine schematische Darstellung eines Depotimplantates in einer weiteren Ausführungsform.

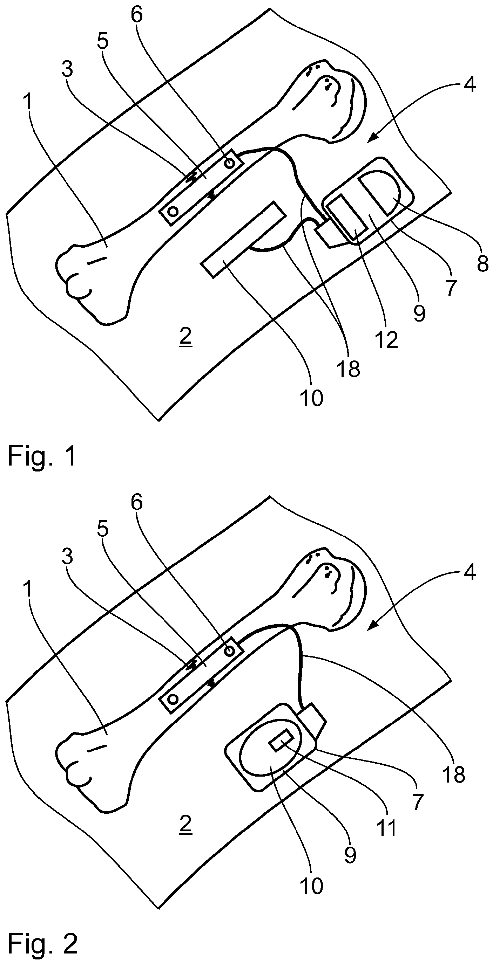

Wie aus Fig. 1 deutlich wird, ist ein Knochen 1 in einer Extremität 2 einer Person mit einer schematisch angedeuteten Fraktur 3 behaftet. Diese ist mit einem als Ganzes mit 4 bezeichneten Implantatsystem medizinisch versorgt, das als Hauptbestandteile eine Knochenschiene 5 mit Befestigungsschrauben 6 als Funktionsimplantat aufweist. Dieses übergreift die Fraktur 3 des Knochens 1 zur Ruhigstellung und Stabilisierung der Knochenfragmente für deren Zusammenwachsen. Die Knochenschiene 5 und Befestigungsschrauben 6 sind aus einem von der Extremität 2 des Körpers des Implantatträgers abbaubaren Metallmaterial, wie beispielsweise Eisen, Magnesium, Zink oder Wolfram bzw. einer geeigneten Legierung dieser Materialien gefertigt.

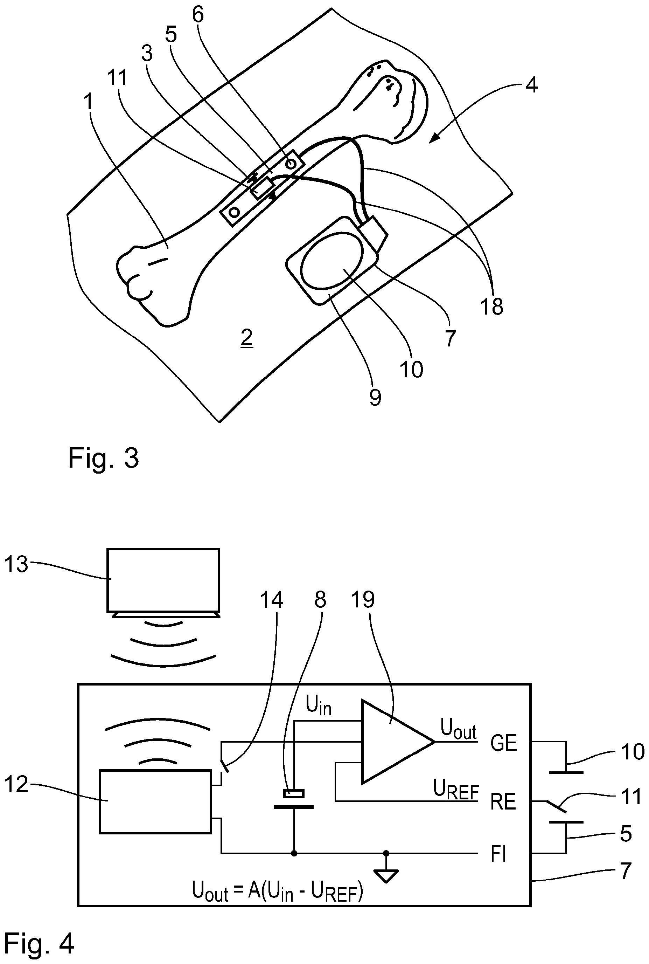

Zweite Hauptkomponente des Implantatsystems ist eine als Ganzes mit 7 bezeichnete Steuereinrichtung, die nach Art eines Herzschrittmachers aufgebaut ist. Zur Energieversorgung ist in die Steuereinrichtung 7 eine Batterie 8 integriert, die in das aus beispielsweise einer Titanlegierung bestehende Gehäuse 9 der Steuereinrichtung 7 integriert ist. Ferner ist eine Gegenelektrode 10 vorgesehen, die aus Platin besteht. Zwischen der Steuereinrichtung 7 einerseits und der Knochenschiene 5 und der Gegenelektrode 10 andrerseits sind Verbindungsleitungen 18 vorgesehen, mit deren Hilfe zwischen der Knochenschiene 5 mit ihren Befestigungsschrauben 6 einerseits und der Gegenelektrode 10 andrerseits eine Polarisationsspannung Uₒᵤₜ (siehe Fig. 4) anlegbar ist. Mithilfe dieser Polarisationsspannung Uₒᵤₜ kann das Degradationsverhalten der Knochenschiene 5 mit ihrer Befestigungsschraube 6 dahingehend gesteuert werden, dass bei Anlegen einer entsprechenden Polarisationsspannung Uₒᵤₜ die Knochenschiene 5 mit den Befestigungsschrauben 6 kathodisch gegen eine Degradation entsprechend der elektrochemischen Spannungsverhältnisse, wie sie anhand der Fig. 4, 6 und 7 erläutert sind, geschützt ist.

Bei den Ausführungsformen gemäß Fig. 2 und 3 ist die Gegenelektrode 10 als Abschnitt des Gehäuses 9 ausgeführt. Ferner ist eine Referenzelektrode 11 vorgesehen, die bei der Variante nach Fig. 2 in die Gegenelektrode 10 isoliert eingebettet ist. Mit der Referenzelektrode 11 kann die am Funktionsimplantat tatsächlich anliegende Spannung U_{REF} ermittelt werden. Das Ermitteln der tatsächlich anliegenden Spannung U_{REF} ist notwendig zur Regelung des Systems - nämlich der zwischen Gegenelektrode 10 und Funktionsimplantat angelegten Spannung Uₒᵤₜ, die den Strom I von der Gegenelektrode10 über das Gewebe zum Funktionsimplantat fließen lässt -, da über den elektrischen Widerstand des Gewebes R ein gewisser Spannungsabfall U_{IR} (IR-Drop) entsteht, der dazu führt, dass Uᵢₙ und U_{REF} nicht gleich sind, weshalb über einen Verstärker 19 die Spannung Uₒᵤₜ nachgeregelt werden muss (siehe Fig. 4). Der Gewebewiderstand ändert sich in der Regel über den Implantationszeitraum, da sich das das Funktionsimplantat und die Gegenelektrode 10 umgebende Gewebe in Laufe der Zeit ändert.

Die Referenzelektrode 11 ist der Gestalt, dass sie in vivo ein konstantes Potential aufweist und keine signifikanten Ströme (außer die, die man zum Messen braucht) über sie fließen. Als Referenzelektrode 11 kommen Materialien wie z.B. Pt, Ptlr, Gold, Kohlenstoff oder dergleichen in Frage.

Die Lage der Referenzelektrode 11 bezüglich des Funktionsimplantats ist bevorzugt räumlich eng gewählt. Ein Ausführungsbeispiel wäre hier ein Ptlr-Blech mit wenigen Quadratmillimetern Fläche, das durch eine Glas- oder Keramikdurchführung durch das Gehäuse 9 direkt in das Steuergerät 7 integriert ist, wie dies Fig. 2 zeigt. Ein weiteres bevorzugtes Ausführungsbeispiel wäre eine als Ptlr-Blech ausgeführte Referenzelektrode 11 mit wenigen Quadratmillimetern Fläche, die mit einer Verbindungsleitung 18 zum Steuergerät 7 direkt auf der Knochenschiene 5 angebracht, z.B. aufgeklebt ist (Fig. 3). Wichtig ist, dass es in beiden Fällen keinen Kurzschluss zur Gegenelektrode 10 bzw. zum Funktionsimplantat 5 gibt.

Neben der Referenzelektrode können auch noch weiter Sensoren verwendet werden. So lassen sich z.B. der pH-Wert und die Temperatur aufzeichnen. Ebenso können Mikroarrays angeschlossen werden, die sensibel auf biologische Faktoren wie Entzündungen reagieren.

Sobald die Degradation der Knochenschiene 5 mit ihren Befestigungsschrauben 6 voranschreiten soll, ist die Polarisationsspannung Uₒᵤₜ zu erniedrigen oder gänzlich auszuschalten. Hierzu genügt es, wenn mithilfe eines kleinen Eingriffs die Verbindungsleitung 18 zwischen Steuereinrichtung 7 und Knochenschiene 5 unterbrochen wird. Soweit sicher ist, dass eine Polarisationsspannung nicht mehr benötigt wird, wird die Steuereinrichtung 7 explantiert.

Wie aus Fig. 4 deutlich wird, erlaubt die Referenzelektrode 11 dabei die Messung der tatsächlich anliegenden Spannung U_{REF}, die mit der gewünschten Spannung Uᵢₙ verglichen wird. Unterscheiden sich U_{REF} und Uᵢₙ, wird die gewünschte Gleichheit durch entsprechende Anpassung der Spannung Uₒᵤₜ mittels des Verstärkers 19 erreicht. Es kann ferner die Steuereinrichtung 7 eine telemetrische Datenübertragungseinheit 12 aufweisen, mit deren Hilfe Messdaten über den Zustand des Funktionsimplantates, also im Wesentlichen der Knochenschiene 5, durch nicht näher dargestellte Sensoren gesammelt und zu einer externen Basiseinheit 13 übertragen werden können. Von dort sind auch Steuerdaten zur Steuerung der von der Batterie 8 erzeugten Spannung Uᵢₙ und damit der Polarisationsspannung Uₒᵤₜ sowohl hinsichtlich deren Wert als auch zeitlichen Verlaufs eingebbar. Entsprechend ist über einen Schalter 14 der Steuereinrichtung 7 die Polarisationsspannung Uₒᵤₜ auch gänzlich abschaltbar. Mit diesen Funktionalitäten ist ein komplettes Home-Monitoring-System realisierbar.

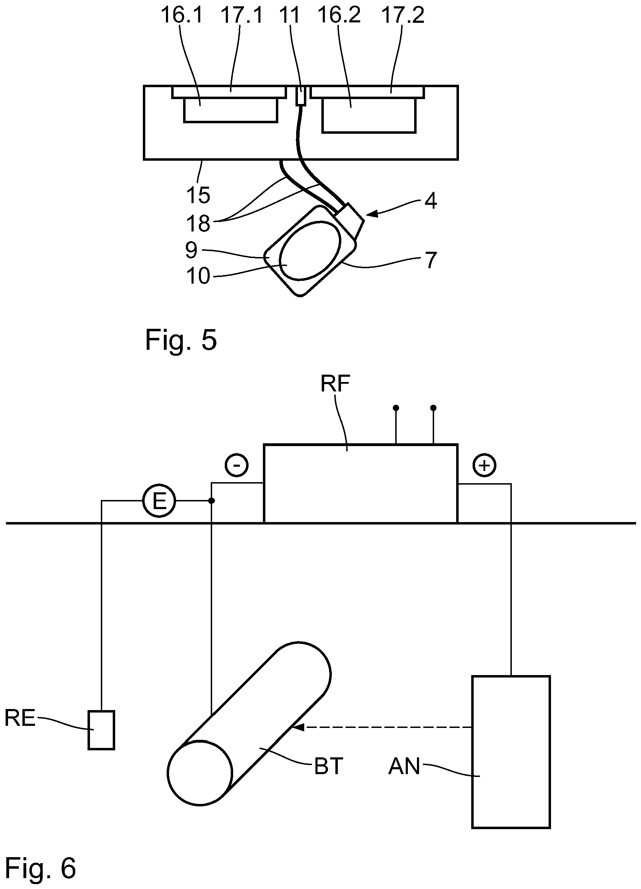

Fig. 5 zeigt eine alternative Ausführungsform des Implantatsystems 4, bei dem als Funktionsimplantat ein Depotimplantat 15 vorgesehen ist, das über mehrere Depotkammern 16.1, 16.2 verfügt. Diese sind jeweils mit unterschiedlichen Wirkstoffen gefüllt und durch eine Abdeckung 17.1, 17.2 aus verschiedenen abbaubaren Metallmaterialien verschlossen. Die Abdeckungen 17.1, 17.2 sind wiederum mit einer Steuereinrichtung 7 der oben anhand von Fig.1 und 2 erläuterten Art verbunden, die zwischen der Gegenelektrode 11 und den Abdeckungen 17.1, 17.2 eine Polarisationsspannung Uₒᵤₜ zur Steuerung des Degradationsverhaltens der Abdeckungen 17.1, 17.2 erzeugen kann. Entsprechend kann die Freisetzung der Wirkstoffe in den Depotkammern 16.1, 16.2 definiert eingestellt werden.

Anhand der Fig. 6 ist das Degradationsverhalten von abbaubaren Metallmaterialien wie folgt zu erläutern:
Kathodischer Korrosionsschutz kann auf zwei Wegen erreicht werden:
a) durch die Verwendung von Opferanoden (Sacrificial Anode) oder
b) durch Außenstrom bzw. Fremdstrom-Anoden (ICCP; Impressed Cathodic Current Protection).

Im Falle von a) verbindet man ein sehr reaktives Hilfsmetall direkt mit dem zu schützenden Metall. Das Hilfsmaterial bildet dabei die Anode des Systems. Die Differenz der freien Korrosionspotentiale zwischen Anode und dem zu schützenden Material verursacht einen von der Anode herkommenden Stromfluss durch den Elektrolyten. Die Potentialdifferenz wird durch die relative Position der verwendeten Metalle in der elektrochemischen Spannungsreihe im verwendeten Elektrolyten bestimmt.

Die in Fig. 6 wiedergegebene Außenstrom-Technik gemäß b) wird meist für den Schutz vergrabener Rohrleitungen und an Schiffsrümpfen benutzt, die Meerwasser ausgesetzt sind. Ein Gleichstromstromkreis wird dabei benutzt, um einen elektrischen Strom an der metallischen Struktur anzulegen. Die Minus-Klemme der Stromquelle RF wird an das Metall angeschlossen, das geschützt werden soll. Die Plus-Klemme wird an eine zusätzliche Anode AN angeschlossen, die sich im gleichen Medium befindet, um den Stromkreis zu schließen. Der elektrische Strom lädt die Struktur mit überschüssigen Elektronen auf und ändert dadurch die Elektrodenspannung in die negative Richtung, bis die Immunitätsregion erreicht ist. Dabei ist es wichtig, dass die Anode AN und die Kathode KA komplett voneinander getrennt sind, damit ein Stromfluss durch das Medium zwischen den Elektroden von der Anode zur Kathode stattfinden kann. Die Funktion der Referenzelektrode RE ist es, die Elektrodenspannung der geschützten Struktur, in diesem Beispielsfall einer vergrabenen Rohrleitung, zu überwachen, um sicherzustellen, dass die Immunitätsregion erreicht wird. Die Referenzelektrode RE ist so ausgelegt, dass sie ein konstantes Potenzial hat und kein Strom über diese fliest. Der Gleichrichter DC-R tritt als das Spannungsversorgungsteil auf und wird, damit das Potenzial der Struktur genug negativ ist, um die Immunitätsregion zu erreichen, was durch die Referenzelektrode RE geregelt wird.

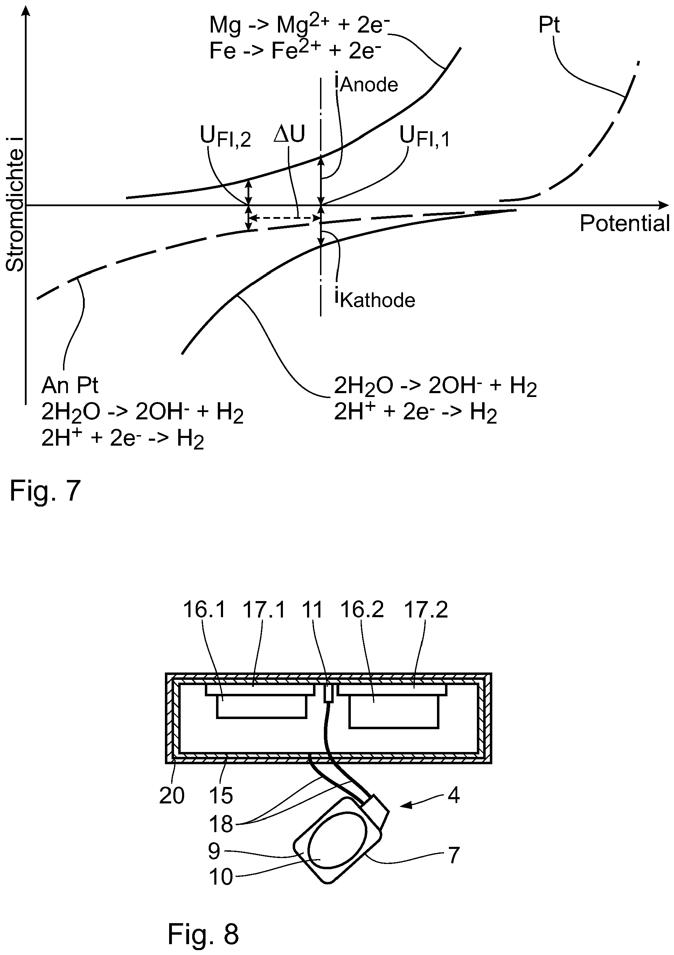

Fig. 7 zeigt ein Depotsystem ähnlich dem in Fig. 5 dargestellten. Insoweit kann auf die dortige Beschreibung verwiesen werden. Im Unterschied dazu ist eine teilweise oder auch vollständige Beschichtung bzw. Ummantelung des gesamten Implantatsystems oder auch nur von Teilen davon vorgesehen. Dazu bieten sich Polymere sowie metallische und keramische Schichten bzw. Schichtsysteme 20 an. Dabei kommen auf dem Funktionsimplantat 15 bevorzugt in vivo abbaubare Beschichtungen bzw. Schichtsysteme 20 zum Einsatz, während auf der Seite des Steuergerätes 7 und der Verbindungsleitungen 18 auch in vivo nicht abbaubare Beschichtungen bzw. Schichtsysteme 20 eingesetzt werden können, damit diese Teile des Implantatsystems später wieder explantiert werden können.

Ferner können die Beschichtungen 20 so ausgelegt werden, dass sie mindestens ein Therapeutikum oder mehrere therapeutische Substanzen oder pharmakologische Wirkstoffe mit einem oder mehreren zeitlichen Verläufen freisetzen können.

## Patentansprüche

1. Implantatsystem umfassend
- ein eine medizinische Funktion erfüllendes Funktionsimplantat (5, 6, 15), insbesondere Knochenimplantat, Gefäßstütze, Stent oder Depotimplantat, das zumindest teilweise aus einem vom Körper (2) des Implantatträgers abbaubaren Metallmaterial gebildet ist, **gekennzeichnet durch**
- eine implantierbare Steuereinrichtung (7) zur Steuerung des Abbauverhaltens des Funktionsimplantats (5, 6, 15) mit
= mindestens einer Gegenelektrode (10) zum Funktionsimplantat (5, 6, 15), und
= einer Spannungsquelle (8) zur Bereitstellung einer Polarisationsspannung (ΔU) zwischen Funktionsimplantat (5, 6, 15) und Gegenelektrode (10) zur Steuerung des Degradationsverhaltens des Funktionsimplantats (5, 6, 15).

2. Implantatsystem nach Anspruch 1, **dadurch gekennzeichnet, dass** mindestens eine Referenzelektrode (11) zur Ermittlung der am Funktionsimplantat (5, 6, 15) tatsächlich anliegenden Spannung (U_{ref}) vorgesehen ist.

3. Implantatsystem nach Anspruch 2, **dadurch gekennzeichnet, dass** die Referenzelektrode (11) aus einem Material ausgewählt aus der Gruppe Platin, Platin-Iridium-Legierung, Gold oder Kohlenstoff besteht.

4. Implantatsystem nach Anspruch 2 oder 3, **dadurch gekennzeichnet, dass** die Referenzelektrode (11) in räumlich enger Nähe zum Funktionsimplantat (5, 6, 15) angeordnet ist.

5. Implantatsystem nach einem der vorgenannten Ansprüche, **dadurch gekennzeichnet, dass** die Steuereinrichtung (7) eine implantierbare Batterie (8) als Spannungsquelle aufweist.

6. Implantatsystem nach einem der vorgenannten Ansprüche, **dadurch gekennzeichnet, dass** die Steuereinrichtung (7) eine separat implantierbare Gegenelektrode (10) aufweist.

7. Implantatsystem nach einem der vorgenannten Ansprüche, **dadurch gekennzeichnet, dass** am Gehäuse (9) der implantierbaren Steuereinrichtung (7) die Gegenelektrode (10) ausbildet ist.

8. Implantatsystem nach einem der vorgenannten Ansprüche, **dadurch gekennzeichnet, dass** die Spannungsquelle (8) durch Trennung der Leitungsverbindung (18) zur Gegenelektrode (10) oder durch Explantierung schaltbar ist.

9. Implantatsystem nach einem der vorgenannten Ansprüche, **dadurch gekennzeichnet, dass** das Funktionsimplantat (5, 6, 15) aus einem oder mehreren Metallmaterialien der Gruppe Eisen, Magnesium, Zink und Wolfram oder deren Legierungen besteht.

10. Implantatsystem nach einem der vorgenannten Ansprüche, **dadurch gekennzeichnet, dass** die Gegenelektrode (10) aus einem Edelmetall, insbesondere Platin, oder einem degradierbaren Metallmaterial besteht, das edler als das Metallmaterial des Funktionsimplantats ist.

11. Implantatsystem nach einem der vorgenannten Ansprüche, **dadurch gekennzeichnet, dass** die Steuereinrichtung (7) eine telemetrische Datenübertragungseinheit (12) aufweist.

12. Implantatsystem nach Anspruch 11, **dadurch gekennzeichnet, dass** über die telemetrische Datenübertragungseinheit (12) Messdaten über den Zustand des Funktionsimplantates (5, 6, 15) und/oder Steuerdaten zur Steuerung der von der Spannungsquelle (8) erzeugten Polarisationsspannung (ΔU) übertragbar sind.

13. Implantatsystem nach einem der vorgenannten Ansprüche, **dadurch gekennzeichnet, dass** das Funktionsimplantat ein Depotimplantat (15) ist, dessen mindestens eine Therapeutikum-gefüllte Depotkammer (16.1, 16.2) zumindest teilweise von einem in seiner Degradation mittels der Steuereinrichtung (7) steuerbaren Metallmaterial gebildet ist.

14. Implantatsystem nach einem der vorgenannten Ansprüche, **dadurch gekennzeichnet, dass** das Funktionsimplantat (5) und/oder die Steuereinheit (7) mit mindestens einer permanenten oder in vivo abbaubaren Beschichtung (20) überzogen ist, aus der mindestens ein Therapeutikum oder pharmakologischer Wirkstoff freisetzbar ist.

15. Implantatsystem nach Anspruch 14 , **dadurch gekennzeichnet, dass** die Beschichtung ein Polymer, ein Metall, eine Legierung oder/und eine Keramik ist.

## Claims

1. Implant system, comprising
- a functional implant (5, 6, 15) that fulfills a medical function, particularly a bone implant, vascular support, stent, or depot implant, which is formed at least in part by a metal material that can be degraded by the body (2) of the implant wearer, **characterized by**
- an implantable control device (7) for controlling the degradation behavior of the functional implant (5, 6, 15), having
= at least one counter-electrode (10) to the functional implant (5, 6, 15), and
= a voltage source (8) for making available a polarization voltage (ΔU) between functional implant (5, 6, 15) and counter-electrode (10), to control the degradation behavior of the functional implant (5, 6, 15).

2. Implant system according to claim 1, **characterized in that** at least one reference electrode (11) for determining the voltage (U_{ref}) actually applied to the functional implant (5, 6, 15) is provided.

3. Implant system according to claim 2, **characterized in that** the reference electrode (11) consists of a material selected from the group of platinum, platinum-iridium alloy, gold, or carbon.

4. Implant system according to claim 2 or 3, **characterized in that** the reference electrode (11) is disposed in close spatial proximity to the functional implant (5, 6, 15).

5. Implant system according to any of the preceding claims, **characterized in that** the control device (7) has an implantable battery (8) as a voltage source.

6. Implant system according to any of the preceding claims, **characterized in that** the control device (7) has a separately implantable counter-electrode (10).

7. Implant system according to any of the preceding claims, **characterized in that** the counter-electrode (10) is configured on the housing (9) of the implantable control device (7).

8. Implant system according to any of the preceding claims, **characterized in that** the voltage source (8) can be switched by means of cutting the line connection (18) to the counter-electrode (10), or by means of explantation.

9. Implant system according to any of the preceding claims, **characterized in that** the functional implant (5, 6, 15) consists of one or more metal materials of the group of iron, magnesium, zinc, and tungsten, or their alloys.

10. Implant system according to any of the preceding claims, **characterized in that** the counter-electrode (10) consists of a precious metal, particularly platinum, or of a degradable metal material that is more precious than the metal material of the functional implant.

11. Implant system according to any of the preceding claims, **characterized in that** the control device (7) has a telemetrical data transmission unit (12).

12. Implant system according to claim 11, **characterized in that** measurement data concerning the status of the functional implant (5, 6, 15) and/or control data for controlling the polarization voltage (ΔU) generated by the voltage source (8) can be transmitted by way of the telemetrical data transmission unit (12).

13. Implant system according to any of the preceding claims, **characterized in that** the functional implant is a depot implant (15) whose at least one depot chamber (16.1, 16.2), filled with a therapeutic agent, is at least partly formed by a metal material whose degradation can be controlled by means of the control device (7).

14. Implant system according to any of the preceding claims, **characterized in that** the functional implant (5) and/or the control unit (7) is/are coated with at least one coating (20) that is permanent or degradable in vivo, from which coating at least one therapeutic agent or pharmacologically active substance can be released.

15. Implant system according to claim 14, **characterized in that** the coating is a polymer, a metal, an alloy or/and a ceramic.

## Revendications

1. Système d'implant comprenant
- un implant fonctionnel (5, 6, 15) accomplissant une fonction médicale, notamment un implant osseux, un support de vaisseau, un stent ou un implant de dépôt, qui est formé au moins partiellement à base d'un matériau métallique dégradable par le corps (2) du porteur d'implant, **caractérisé par**
- un dispositif de commande (7) implantable pour la commande du comportement de dégradation de l'implant fonctionnel (5, 6, 15) avec
= au moins une contre-électrode (10) par rapport à l'implant fonctionnel (5, 6, 15), et
= une source de tension (8) pour l'établissement d'une tension de polarisation (ΔU) entre l'implant fonctionnel (5, 6, 15) et la contre-électrode (10) pour la commande du comportement de dégradation de l'implant fonctionnel (5, 6, 15).

2. Système d'implant selon la revendication 1, **caractérisé en ce qu'**au moins une électrode de référence (11) est prévue pour la détermination de la tension (U_{ref}) appliquée effectivement sur l'implant fonctionnel (5, 6, 15).

3. Système d'implant selon la revendication 2, **caractérisé en ce que** l'électrode de référence (11) est constituée d'un matériau choisi dans le groupe du platine, d'un alliage de platine et d'iridium, de l'or ou du carbone.

4. Système d'implant selon la revendication 2 ou 3, **caractérisé en ce que** l'électrode de référence (11) est disposée à proximité de l'implant fonctionnel (5, 6, 15).

5. Système d'implant selon l'une des revendications précitées, **caractérisé en ce que** le dispositif de commande (7) présente une pile (8) implantable en tant que source de tension.

6. Système d'implant selon l'une des revendications précitées, **caractérisé en ce que** le dispositif de commande (7) présente une contre-électrode (10) implantable séparément.

7. Système d'implant selon l'une des revendications précitées, **caractérisé en ce que** la contre-électrode (10) est formée sur le boîtier (9) du dispositif de commande (7) implantable.

8. Système d'implant selon l'une des revendications précitées, **caractérisé en ce que** la source de tension (8) peut être activée par une séparation de la connexion de ligne (18) vers la contre-électrode (10) ou par une explantation.

9. Système d'implant selon l'une des revendications précitées, **caractérisé en ce que** l'implant fonctionnel (5, 6, 15) est constitué d'un ou de plusieurs matériaux métalliques du groupe du fer, du magnésium, du zinc et du tungstène ou de leurs alliages.

10. Système d'implant selon l'une des revendications précitées, **caractérisé en ce que** la contre-électrode (10) est constituée d'un métal noble, notamment de platine, ou d'un matériau métallique dégradable qui est plus noble que le matériau métallique de l'implant fonctionnel.

11. Système d'implant selon l'une des revendications précitées, **caractérisé en ce que** le dispositif de commande (7) présente une unité de transmission de données (12) télémétrique.

12. Système d'implant selon la revendication 11, **caractérisé en ce que** des données de mesure concernant l'état de l'implant fonctionnel (5, 6, 15) et/ou des données de commande pour la commande de la tension de polarisation (ΔU) générée par la source de tension (8) peuvent être transmises par le biais de l'unité de transmission de données (12) télémétrique.

13. Système d'implant selon l'une des revendications précitées, **caractérisé en ce que** l'implant fonctionnel est un implant de dépôt (15) dont l'au moins une chambre de dépôt (16.1, 16.2) remplie d'un produit thérapeutique est formée au moins partiellement à base d'un matériau métallique réglable dans sa dégradation au moyen du dispositif de commande (7).

14. Système d'implant selon l'une des revendications précitées, **caractérisé en ce que** l'implant fonctionnel (5) et/ou l'unité de commande (7) sont revêtus avec au moins un revêtement (20) permanent ou dégradable in vivo, à partir duquel au moins un produit thérapeutique ou une matière active pharmaceutique peuvent être libérés.

15. Système d'implant selon la revendication 14, **caractérisé en ce que** le revêtement est un polymère, un métal, un alliage et/ou une céramique.
